(19) Europäisches Patentamt
European Patent Office
Office européen des brevets

(11) Publication number: 0 137 487 B1

(12) # EUROPEAN PATENT SPECIFICATION

(45) Date of publication of patent specification: **28.03.90**

(51) Int. Cl.$^5$: **A 61 B 6/02,** G 01 N 23/18, G 01 T 1/24

(21) Application number: **84112101.5**

(22) Date of filing: **09.10.84**

(54) **Energy separated quantum-counting radiography and apparatus.**

(30) Priority: **12.10.83 JP 189197/83**

(43) Date of publication of application:
**17.04.85 Bulletin 85/16**

(45) Publication of the grant of the patent:
**28.03.90 Bulletin 90/13**

(84) Designated Contracting States:
**DE FR GB**

(56) References cited:
**DE-A-2 544 354**
**DE-A-2 753 004**
**GB-A-2 082 873**
**US-A-3 974 386**
**US-A-4 029 963**
**US-A-4 284 895**

**MEDICA MUNDI, vol. 27, no. 3, 1982, pages 125-132; J. COUMANS et al.: "Computerized dual-energy imaging; a technical description"**

(73) Proprietor: **Matsushita Electric Industrial Co., Ltd.**
**1006, Oaza Kadoma**
**Kadoma-shi Osaka-fu, 571 (JP)**

(72) Inventor: **Baba, Sueki**
**19-116, Izumi-cho 2-chome**
**Suita City 564 (JP)**
Inventor: **Yamamoto, Osamu**
**9-2, Keihan-Kitahondouri**
**Moriguchi City 570 (JP)**
Inventor: **Yamashita, Tadaoki**
**16-1, Inda-cho**
**Kirakata City 573 (JP)**
Inventor: **Tsutsui, Hiroshi**
**39-118, Hashimoto-Kurigatani**
**Yawata City 614 (JP)**

(74) Representative: **Kirschner, Klaus Dieter et al**
**Patentanwälte Kirschner & Grosse Forstenrieder Allee 59**
**D-8000 München 71 (DE)**

**EP 0 137 487 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

Courier Press, Leamington Spa, England.

## Description

The present invention relates generally to an energy separated quantum-counting radiography in diagnosis field and industrial field and so on.

Hitherto, there are proposals on recording, examination or observation with X-ray or the like radiography for obtaining two dimensional image. The conventional radiography has such disadvantages that considerable complicated operation is necessary for obtaining a radiation image having informations of composition or density of a complicated material of an object body.

The projection of a quantum-counting radiography using a gaseous chamber and a crystal scintillator followed by a photo-detector is known from US—A—40 29 963. Here the basis for the measurement are the detector spectra at the low and high energy regions which spectra correspond to a photo-electric component forming an atomic number dependent image and the compton scattering component respectively which compton component is proportional to the electron density. Both these detector spectra are used to calculate the image.

This method is rather cumbersome but it has to be carried out because the single measurements always depend on the photo-electric effect *and* the compton scattering effect.

Actually, the US—A—40 29 963 uses both effects but, admittedly, a rather complicated method to get to a sufficient pulse energy discrimination over the broad range of X-rays from about 20 KeV to 150 KeV.

### Summary of the Invention

The purpose of the present invention is to provide a superior quantum-counting radiography which can detect in real-time a material or a density distribution of the object body on the basis of the information mainly with regard to the amounts of the radiation rays having penetrated through the object body, the amounts being discriminated every radiation ray energy group, thereby to dissolve the disadvantages of the conventional radiography.

The energy separated quantum-counting radiography of the present invention comprises the steps defined in independent method claim 5.

Further, the energy separated quantum-counting radiographic apparatus of the present invention comprises the features defined in independent apparatus claim 1.

### Brief Description of the Drawing

Fig. 1(a) is a perspective view showing outline configuration of the energy separated quantum-counting radiography.

Fig. 1(b) is a circuit diagram of a radiation sensitive elements array and other circuits of the energy separated quantum-counting radiography shown in Fig. 1(a).

Fig. 2(a) is a graph showing a pulse wave height spectrum distribution with regard to almost uniform energy X-ray which has not penetrated yet through an object body.

Fig. 2(b) is a graph showing a pulse wave height spectrum distribution with regard to the uniform energy X-ray which has penetrated through the object body.

Fig. 3(a) is a graph showing a pulse wave height spectrum distribution with regard to a uniform energy X-ray which has not penetrated through the object body.

Fig. 3(b) is a graph showing a pulse wave height spectrum distribution with regard to another uniform energy X-ray which has not penetrated through the object body.

Fig. 3(c) is a graph showing a pulse wave height spectrum distribution with regard to two kinds of X-rays having two energies shown in Figs. 3(a) and 3(b), which have penetrated through the object body.

Fig. 4 is a graph showing characteristics between mass absorption coefficient and X-ray energy with regard to main object body materials.

Fig. 5 is a graph showing characteristics between linear absorption coefficient of compton effect and photo-electric effect with regard to various kinds of radiation sensitive semiconductor elements.

### Description of Preferred Embodiment

Fig. 1(a) is a perspective view of one embodiment for showing a principle of an energy separated quantum-counting radiography.

An object body 2 such as a human body is disposed between a radiation source 1, for example, of an X-ray source and an array 3 of radiation sensitive elements 5, 5, .... A circuit 4 for processing signals issued from the radiation sensitive elements 5, 5, ... of the array 3, is connected to each radiation sensitive elements 5, 5, ... of the array 3.

Radiation rays having plural kinds of energies or wide energy band are emitted from slits of the radiation source 1 radially against the array 3 of the radiation sensitive elements 5, 5, .... The radiation ray penetrates through the object body 2. The linear or arc-shaped array 3 of the radiation sensitive elements 5, 5, ... receives the radiation rays having penetrated through the object body, to detect the amount of the radiation rays as pulse number. The radiation source 1 and the array 3 of the radiation sensitive elements 5, 5, ... are transferred from upper position to lower position at a constant velocity, thereby to produce a whole radiation image of the object body 2. In place of transferring the array 3, a very large number of arrays 3, 3, ... may be disposed longitudinally.

Fig. 1(b) is a circuit diagram showing details of the array 3 of the radiation sensitive elements 5, 5, ... and the circuit 4. The radiation sensitive elements 5 is made of a semiconductor detector such as Si, GaAs, CdTe. An electric power source 6 supplies a voltage to the radiation sensitive element 5 for driving it. A pulse amplifier 7 is connected to each radiation sensitive element 5 respectively, wherein a FET is used at input part of the pulse amplifier 7, thereby to form a high input impedance circuit. A pulse wave height discriminator 8 is connected to each pulse amplifier 7 respectively. The pulse wave height discriminator 8 comprises plural voltage comparators 8a, 8a, ... to each of which the pulse signal issued from the pulse amplifier 7, is supplied. A reference voltage producing circuit 9 produces various reference voltage signals corresponding to predetermined various wave heights of pulse. The pulse wave heights are selected corresponding to respective energies of the radiation rays. The various reference voltage signals are supplied to each voltage comparator 8a respectively as shown in Fig. 1(b). The voltage comparator 8a compares the pulse wave height, corresponding to the radiation ray energy, of the pulse signal issued from the pulse amplifier 7 with the reference voltage signal corresponding to a predetermined wave height of pulse supplied into the voltage comparator 8a, thereby to issue only such pulse that the pulse wave height of the received pulse is larger than the reference voltage. Thus the pulse wave height discriminator 8 discriminates the pulse wave height of the amplified pulse, thereby to produce pulses grouped in accordance with classified ranges of pulse wave heights.

Pulse counters 10, 10, ... for counting for a predetermined time numbers of pulses issued from the respective voltage comparators 8a, 8a, ... and memorizing the number, are connected to the voltage comparators 8a, 8a, ... respectively. The counted data of respective pulse counters 10, 10, ... are transmitted speedily by a data transmitter 11 to a computer 13. A control circuit 12 controls the data transmitter 11 and the reference voltage producing circuit 9.

The operation and effect of the above-mentioned embodiment is as follows.

According to the above-mentioned embodiment, the amount of the radiation rays having penetrated through the object body is detected as pulse number in a manner that the quantum of the radiation rays gets into a depletion layer of the semiconductor element 5, thereby to obtain a high sensitive and high resolutive radiation image at a high speed. Further, an analysis of the composition and density of the various materials of the object body 2 can be executed at a time as mentioned below.

The principle of the analysis is that the object body 2 is disposed in even radiation ray field and the radiation ray image can be obtained by detecting an unevenness of the penetrating radiation ray, the unevenness being produced by the shielding of the object body 2. The shielding is caused by an absorption of the materials of the object body 2. The absorption amount is determined by the kind of the radiation ray, the kind of the energy of the radiation ray and the kind, amount of the material of the object body. Therefore, the kind and amount of the materials of the object body can be analized by simultaneously irradiating the radiation rays having various energy and detecting the absorption amount, namely, pulse number corresponding to the amount of the radiation ray having penetrated, for each kind of energy of the radiation ray, namely each pulse wave height. As a result, a high sensitive and resolutive radiation image can be speedily obtained. In this embodiment, the absorption amount is obtained based on the number of the pulse issued from the radiation sensitive element 5.

Fig. 2(a) shows the pulse wave height spectrum distribution of X-ray having not penetrated, obtained from the pulse amplifier 7 in case utilizing an X-ray having a uniform energy.

Fig. 2(b) shows the pulse wave height spectrum distribution of the radiation ray having penetrated, obtained from the pulse amplifier 7 in case utilizing an X-ray having a uniform energy.

The pulse wave height spectrum distribution in Fig. 2(a) has certain narrow width with the pulse wave height $H_A$ as the central position and has a peak value $I_A$ of the number of pulse.

The pulse wave height spectrum distribution in Fig. 2(b) has certain narrow width with the pulse wave height $H_A$ as the central position as same as in Fig. 2(a) but has a peak value $O_A$ of the number of pulse which is lower than the peak value $I_A$ in Fig. 2(a).

The correlation of the peak value $O_A$ and the peak value $I_A$ is shown by the next formula (1).

$$O_A = I_A \{1 - e^{-(\Sigma \mu_n x_n)}\} \qquad (1)$$

wherein $\mu_n$ is linear absorption coefficient of material of the object body [$cm^{-1}$] and $x_n$ is virtual thickness [cm] of the object body having $\mu_n$.

In this embodiment, in place of the peak value $I_A$, an integrated value $T_{AI}$ as shown by hatched lines in Fig. 2(a), is used, which is obtained by integrating total number of pulse having larger pulse wave height than a smallest pulse wave height A in order to avoid noise such as drift of the circuit. Likewise, in place of the peak value $O_A$, an integrated value $T_{AO}$ as shown by hatched lines in Fig. 2(b) is used, which is obtained by integrating total number of pulse having larger wave height than a smallest pulse wave height A. The integrating operation is executed in the counter 10.

The correlation of the integrated value $T_{AI}$ and the integrated value $T_{AO}$ is shown by the next formula (2).

$$T_{AO} = T_{AI} \{1 - e^{-(\Sigma \mu_n x_n)}\} \qquad (2)$$

The pulses having smaller pulse wave height than the pulse wave height A includes various noises and the pulses can be avoided by the pulse wave height discriminator 8, therefore the deterioration of the radiation image caused by the noise can be prevented in the present invention.

Fig. 3(c) shows pulse wave height spectrum distribution of the X-ray having penetrated, obtained from the pulse amplifier 7 when two kinds of X-ray having different energy as shown in Figs. 3(a) and 3(b) are emitted simultaneously from the radiation source 1. The energy of the X-ray is corresponding to the wave height of the pulse.

The pulse signal issued from the pulse amplifier 7 is supplied to each plural voltage comparators 8a of the pulse wave height discriminator 8. Each voltage comparator 8a compares the pulse wave height of the pulse signal with reference signal thereof and issues only such pulses that the pulse wave height is larger than the reference voltage. The pulse wave height discriminator 8 can discriminate simultaneously the pulse signals having various pulse wave heights as shown in Fig. 3(c) into some groups each having certain pulse wave height range, for example, a group of wave height A to B and another group of wave height B to C. Therefore, various information with regard to the X-ray can be obtained by one irradiation of the X-ray.

Figs. 3(a), (b) and (c) relate to two kinds of energies of X-rays but the kinds of energies of X-rays is not limited into two kinds of energies. The number of kinds of energies of X-rays can be increased so as to be able to separate the wave height group each other.

Thus the classified pulse signals are supplied to respective pulse counter 10 from the voltage comparators 8a, 8a, . . . and the pulse counters 10, 10, . . . count the numbers of the respective groups of pulses and memorize the numbers and the numbers are transmitted to the computer 13. The difference between the number of pulses of the X-ray having not penetrated and the number of pulses of the X-ray having penetrated is corresponding to the absorption amount of the X-ray. Therefore, absorption amount which is classified according to the kind of energy of the X-ray can be produced by the computer 13. The computer 13 further produces the kind or the amount of the material of the object body by utilizing the characteristics between mass absorption coefficient ($cm^2/g$) and X-ray energy with regard to main object body materials.

Fig. 4 shows a characteristics between mass absorption coefficient ($cm^2/g$) and X-ray energy (KeV) with regard to main object body materials. There is very large difference of the mass absorption coefficient among a muscle, bone, calcium (Ca) and iodine (I) which are used as contrast media in the energy region from 30 KeV to 150 KeV. That is, for example, the mass absorption coefficient of the bone is twice as large as that of the muscle at 40 KeV which is corresponding to a certain pulse wave height group, but the mass absorption coefficient of the bone is almost as same as that of the muscle at 150 KeV which is corresponding to another pulse wave height group. Therefore, a radiation image of bone or a radiation image of muscle can be obtained at one time by utilizing a simultaneous linear equations etc., based on the counted number of the energy groups and the characteristics in Fig. 4. Further, a quantitative detection of the bone or the muscle etc. can be executed. Thus, the energy separated quantum-counting radiography of the present invention can realise a more speedy and more resolutive radiation image radiography than other radiation image radiographies using a subtraction method.

Fig. 5 shows a characteristics between the linear absorption coefficient of compton effect and photo-electric effect with regard to various kinds of X-ray radiation sensitive semiconductor elements. The present invention mainly uses the photo-electric effect, since the pulse output based on the compton absorption does not have high correlation between the input energy and the pulse output. The compton absorption produces a noise of pulse count. Therefore, the present invention uses such materials as the semiconductor, that the photo-electric absorption effect is larger than the compton absorption effect from a general medical examination energy region to a non-destructive detecting energy region.

The kinds of the radiation rays of the present invention are a combination of an X-ray tube and a filter which can vary the energy of the X-ray, a specific X-ray beam and Radio Isotope (RI) of $^{241}Am$, Ir, etc. An electron beam which is independent to atomic number can be used with the X-ray together. Further a γ-beam, a neutron-beam or a meson-beam can be used as the radiation ray. The above-mentioned kinds of radiation rays can be used as any combination.

The radiation source 1 can irradiate the radiation ray continually or intermittently. Further the radiation source 1 can irradiate each kind of energy radiation ray or each kind of radiation ray alternately or simultaneously in case that said radiation rays are plural kinds of radiation rays or plural kinds of energy radiation rays.

As above-mentioned, the energy separated quantum-counting radiography of the present invention has the following advantages not known in the prior art.

That is, the energy separated quantum-counting radiography of the present invention uses a pulse counting method, therefore the energy separated quantum-counting radiography can obtain a high sensitive and high resolutive radiation image. Further the energy separated quantum-counting radiography uses such method counting pulses for each pulse wave height group, therefore the radiation image having the information of the kinds of the composition materials of the object body can be obtained speedily.

Further the energy separated quantum-counting radiography can be applied to a computer tomography, to obtain more quantitative information of the composition materials of the object body. The amount of the radiation rays radiated against the object body, for example human body, can be decreased since the energy separated quantum-counting radiography can be executed in one radiation operation.

**Claims**

1. An energy separated quantum-counting radiography method comprising the steps of
emitting radiation rays from a radiation source,
receiving radiation rays which penetrate through an object body by a linear or arc-shaped array of radiation sensitive elements and moving continually or intermittently relative spatial relation of said radiation source and/or said radiation sensitive elements with respect to said object body, thereby to induce signals containing radiographic information,
said radiation sensitive elements simultaneously receiving said radiation rays to detect said radiation ray quantum pulses,
emitting radiation rays having plural kinds of energies or a wide energy band,
amplifying said pulses issued from said radiation sensitive elements,
discriminating pulse wave heights of said amplified pulses, thereby to produce pulses classified into groups by ranges of pulse wave height,
counting for a predetermined time each number of said pulses belonging to respective groups, and
obtaining a radiation image information by utilizing said pulses belonging to respective groups, and
obtaining a radiation image information by utilizing each counted number of said pulses belonging to respective groups,
characterized in that
said radiation sensitive elements are semiconductor elements, wherein the photo-electric absorption effect is larger than the compton absorption effect.

2. An energy separated quantum-counting radiography method in accordance with claim 1, wherein
obtaining another each counted number of said pulses belonging to respective groups, with the object body being eliminated,
calculating an attenuation amount of said radiation rays by comparing said each counted number of said pulses belonging to respective groups in case that said object body exists, with said corresponding another each counted number of said pulses belonging to respective groups in case that said object body does not exist,
obtaining a distribution image of a chemical element of said object body or a distribution image of a density of said object body.

3. An energy separated quantum-counting radiography method in accordance with claim 1 or 2, wherein
said radiation source radiates one or plural kinds of radiation rays selected among an X-ray, a γ-beam, a β-beam, a neutron-beam and a meson-beam,
each radiation ray can have one kind of energy in case that said radiation rays are plural kinds.

4. An energy separated quantum-counting radiography method in accordance with claim 1 or 2, wherein
said radiation source irradiates one or plural kinds of radiation rays selected among an X-ray, a γ-beam, a β-beam, a neutron-beam and a meson-beam,
each radiation ray can have a kind of energy in case that said radiation rays are plural kinds,
said radiation source irradiates continually or intermittently said radiation rays,
said radiation source irradiates each kind of energy radiation ray or each kind of radiation ray alternately or simultaneously in case that said radiation rays are plural kinds of radiation rays or plural kinds of energy radiation rays.

5. An energy separated quantum-counting radiographic apparatus comprising
a radiation source for emitting radiation rays, having plural kinds of energy or a wide energy band,
a linear or arc-shaped array of radiation sensitive elements for receiving radiation rays which penetrate through an object body and for detecting said radiation as pulses,
said radiation source and/or said radiation sensitive elements being translated relatively against said object body, thereby a radiation image of said object being obtained,
a pulse amplifier connected to each radiation sensitive element for producing amplified pulses,
a pulse wave height discriminator for discriminating the pulse wave heights of said amplified pulses, thereby to produce pulses classified into groups by ranges of pulse wave height,
a pulse counter for counting for a predetermined time each number of said pulses belonging to respective groups, and
a computer for receiving each number counted by said pulse counter, thereby to obtain a radiation image of said object body,
characterized in that
said radiation sensitive elements are semiconductor elements, wherein the photo-electric absorption effect is larger than the compton absorption effect.

6. An energy separated quantum-counting radiographic apparatus in accordance with claim 5, wherein
said radiation source irradiates one or plural kinds of radiation rays selected among an X-ray, a γ-beam, a β-beam, a neutron-beam and a meson-beam,
each radiation ray can have one kind of energy in case that said radiation rays are plural kinds.

7. An energy separated quantum-counting radiographic apparatus in accordance with claim 5, wherein
said radiation source irradiates one or plural kinds of radiation rays selected among an X-ray, a γ-beam, a β-beam, a neutron-beam and a meson-beam,
each radiation ray can have a kind of energy in case that said radiation are plural kinds,
said radiation source irradiates continually or intermittently said radiation rays,

said radiation source irradiates each kind of energy radiation ray or each kind of radiation ray alternately or simultaneously in case that said radiation rays are plural kinds of radiation rays or plural kinds of energy radiation rays.

**Patentansprüche**

1. Strahlungsabbildungsverfahren mit nach Energieklassen getrennter Quantenzählung, welches folgende Schritte umfaßt:

Aussenden von Strahlung von einer Strahlungsquelle,

Auffangen von Strahlung, die einen Objektkörper durchdringt, durch eine lineare oder bogenförmige Anordnung von strahlungsempfindlichen Elementen und ununterbrochene oder absatzweise Bewegung der Strahlungsquelle und/oder der strahlungsempfindlichen Elemente in räumlicher Beziehung zu dem Objektkörper, um dadurch radiographische Information aufweisende Signale zu induzieren,

gleichzeitiges Auffangen der Strahlung durch die strahlungsempfindlichen Elemente, um die Strahlungsquantenimpulse zu erfassen,

Aussenden von Strahlung mehrerer Energiearten oder eines breiten Energiebandes,

Verstärken der von den strahlungsempfindlichen Elementen abgegebenen Impulse,

Unterscheiden von Impulswellenhöhen der verstärkten Pulse, um dadurch Impulse zu erzeugen, die nach Impulswellenhöhenbereichen in Gruppen eingeteilt werden,

Zählen der jeweiligen Zahl von zu einer jeweiligen Gruppe gehörenden Impulse während eines bestimmten Zeitraums, und

Erhalten eines Strahlungsinformationsabbildes durch Verwendung der zu den jeweiligen Gruppen gehörenden Impulse, und

Erhalten eines Strahlungsinformationsabbildes durch Verwendung jeder gezählen Anzahl von zu den jeweiligen Gruppen gehörenden Impulsen, dadurch gekennzeichnet, daß

die strahlungsempfindlichen Elemente Halbleiterelemente sind, bei denen der Effekt der photoelektrischen Absorption größer ist als der Effekt der Compton-Absorption.

2. Strahlungsabbildungsverfahren mit nach Energieklassen getrennter Quantenzählung nach Anspruch 1, umfassend

Erhalten einer jeweils anderen gezählten Anzahl von zu jeweiligen Gruppen gehörenden Impulsen, wenn das Objekt entfernt ist,

Berechnen eines Dämpfungswertes durch Vergleichen der für den Fall, daß der Objektkörper existiert, jeweils gazählten Anzahl von zu jeweiligen Gruppen gehörenden Impulsen mit der entsprechenden jeweils anderen für den Fall, daß der Objektkörper nicht existiert, gezählten Anzahl von zu den jeweiligen Gruppen gehörenden Impulsen,

Erhalten eines Verteilungsbildes eines chemischen Elementes des Objektkörpers oder eines Verteilungsbildes der Dichte des Objektkörpers.

3. Strahlungsabbildungsverfahren mit nach Energieklassen getrennter Quantenzählung nach Anspruch 1 oder 2, wobei

die Strahlungsquelle eine oder mehrere Arten von Strahlen, Röntgenstrahlen, Gammastrahlen, Betastrahlen, Neutronenstrahlen und Mesonenstrahlen, aussendet,

jeder Strahl für den Fall, daß die Strahlen von unterschiedlicher Art sind, eine Art von Energie haben kann.

4. Strahlungsabbildungsverfahren mit nach Energieklassen getrennter Quantenzählung nach Anspruch 1 oder 2, wobei

die Strahlungsquelle eine oder mehrere Arten von Strahlen, Röntgenstrahlen, Gammastrahlen, Betastrahlen, Neutronenstrahlen und Mesonenstrahlen, aussendet,

jeder Strahl für den Fall, daß die Strahlen von unterschiedlicher Art sind, eine Art von Energie haben kann,

die Strahlungsquelle ununterbrochen oder in Abständen Strahlung abgibt,

die Strahlungsquelle für den Fall, daß die Strahlen mehrere Arten von Strahlen oder mehrere Arten von Energiestrahlen sind, jede Art von Energiestrahlung oder jede Art von Strahlung im Wechsel oder gleichzeitig abgibt.

5. Gerät zur Strahlungsabbildung mit nach Energieklassen getrennter Quantenzählung, umfassend:

eine Strahlungsquelle zum Aussenden von Strahlung mit mehreren Energiearten und einem breiten Energieband,

eine lineare oder bogenförmige Anordnung von strahlungsempfindlichen Elementen zum Auffangen von einen Objektkörper durchdringende Strahlung und zum Erfassen der Strahlung in Form von Impulsen,

wobei die Strahlungsquelle und/oder die strahlungsempfindlichen Elemente bezüglich dem Objektkörper verschoben werden, und dadurch wird ein Strahlungsabbild des Objekts erhalten,

einen mit jedem strahlungsempfindlichen Element verbundenen Impulsverstärker zur Erzeugung verstärkter Impulse,

einen Impulswellenhöhendiskriminator zur Unterscheidung der Impulswellenhöhen der verstärkten Impulse, um dadurch Impulse zu erzeugen, die in Gruppen von Impulswellenhöhenbereichen eingeteilt werden,

einen Impulszähler zum Zählen der Anzahl von zu einer jeweiligen Gruppe gehörenden Impulsen während einer vorgegebenen Zeitdauer, und

einen Rechner zur Aufnahme jeder durch den Impulszähler gezählten Zahl, um dadurch eine Strahlungsabbild des Objektkörpers zu erhalten, dadurch gekennzeichnet, daß

die strahlungsempfindlichen Elemente Halbleiterelemente sind, bei denen der Effekt der photoelektrischen Absorption größer ist als der Effekt der Compton-Absorption.

6. Gerät zur Strahlungsabbildung mit nach Energieklassen getrennter Quantenzählung nach Anspruch 5, wobei

die Strahlungsquelle eine oder mehrere Arten von Strahlen, Röntgenstrahlen, Gammastrahlen, Betastrahlen, Neutronenstrahlen und Mesonenstrahlen, aussendet,

jeder Strahl für den Fall, daß die Strahlen von unterschiedlicher Art sind, eine Art von Energie haben kann.

7. Gerät zur Strahlungsabbildung mit nach Energieklassen getrennter Quantenzählung nach Anspruch 5, wobei

die Strahlungsquelle eine oder mehrere Arten von Strahlen, Röntgenstrahlen, Gammastrahlen, Betastrahlen, Neutronenstrahlen und Mesonenstrahlen, aussendet,

jeder Strahl für den Fall, daß die Strahlen von unterschiedlicher Art sind, eine Art von Energie haben kann,

die Strahlungsquelle ununterbrochen oder in Abständen Strahlung abgibt,

die Strahlungsquelle für den Fall, daß die Strahlen mehrere Arten von Strahlen oder mehrere Arten von Energiestrahlen sind, jede Art von Energiestrahlung oder jede Art von Strahlung im Wechsel oder gleichzeitig abgibt.

**Revendications**

1. Procédé de radiographie par comptage de quanta classés par énergie, comprenant les étapes consistant à:

émettre des rayons d'un rayonnement à partir d'une source de rayonnement,

recevoir des rayons de rayonnement qui pénètrent un corps-objet par un réseau linéaire ou en forme d'arc d'éléments sensibles à un rayonnement et modifier continuellement ou par intermittence la relation spatiale relative de la source de rayonnement et/ou des éléments sensibles au rayonnement par rapport au corps-object, d'où la création de signaux contenant une information radiographique,

les éléments sensibles au rayonnement recevant simultanément les rayons de rayonnement afin de détecter les impulsions quantiques du rayon de rayonnement,

émettre des rayons de rayonnement ayant plusieurs sortes d'énergie ou un large intervalle d'énergie,

amplifier les impulsions émises par les éléments sensibles au rayonnement,

faire une distinction entre les hauteurs des ondes pulsées des impulsions amplifiées, d'où la production d'impulsions classées en groupe par gammes de hauteur des ondes pulsées,

compter pendant un temps prédéterminé chaque nombre d'impulsions appartenant à des groupes respectifs, et

obtenir une information par image du rayonnement en utilisant les impulsions appartenant à des groupes respectifs, et

obtenir une information par image du rayonnement en utilisant chaque nombre compté des impulsions appartenant à des groupes respectifs, caractérisé en ce que:

les éléments sensibles au rayonnement sont des éléments en semi-conducteur, où l'effet d'absorption photoélectrique est supérieur à l'effet d'absorption Compton.

2. Procédé de radiographie par comptage de quanta classés par énergie selon la revendication 1, caractérisé par les étapes consistant à:

obtenir un autre nombre compté des impulsions appartenant à des groupes respectifs, avec le corps-objet éliminé,

calculer une quantité d'atténuation des rayons de rayonnement en comparant chaque nombre compté des impulsions appartenant à des groupes respectifs dans le cas où le corps-objet existe, à un autre nombre compté correspondant des impulsions appartenant à des groupes respectifs dans le cas où le corps-objet n'existe pas,

obtenir une image de la distribution d'un élément chimique du corps-objet ou une image de la distribution de la densité du corps-objet.

3. Procédé de radiographie par comptage de quanta classés par énergie selon la revendication 1 ou 2, dans lequel:

la source de rayonnement émet une ou plusieurs sortes de rayons de rayonnement choisis parmi les rayons X, un faisceau γ, un faisceau β, un faisceau de neutrons et un faisceau de mesons,

chaque rayon de rayonnement peut avoir une sorte d'énergie dans le cas où les rayons de rayonnement sont de plusieurs sortes.

4. Procédé de radiographie par comptage de quanta classés par énergie selon la revendication 1 ou 2, et dans lequel:

la source de rayonnement émet une ou plusieurs sortes de rayons de rayonnement choisis parmi les rayons X, un faisceau γ, un faisceau β, un faisceau de neutrons et un faisceau de mesons,

chaque rayon de rayonnement peut avoir une sorte d'énergie dans le cas où le rayonnement est de plusieurs types,

la source de rayonnement émet continuellement ou par intermittence les rayons de rayonnement,

la source de rayonnement émet chaque sorte de rayons de rayonnement d'énergie ou chaque sorte de rayons de rayonnement alternativement ou simultanément dans le cas où les rayons de rayonnement sont plusieurs sortes de rayons de rayonnement ou plusieurs sortes de rayons de rayonnement d'énergie.

5. Appareil radiographique à comptage de quanta classés par énergie, comprenant:

une source de rayonnement pour émettre des rayons de rayonnement, ayant plusieurs sortes d'énergie ou un large intervalle d'énergie,

un réseau linéaire ou en forme d'arc d'éléments sensibles à un rayonnement pour recevoir les rayons de rayonnement qui pénètrent un corps-objet et pour détecter le rayonnement sous forme d'impulsions,

la source de rayonnement et/ou les éléments sensibles au rayonnement étant l'objet d'une translation relative contre le corps-objet, d'où l'obtention d'une image de l'objet par rayonnement,

un amplificateur d'impulsions relié à chaque élément sensible de rayonnement pour produire des impulsions amplifiées,

un discriminateur de hauteur d'ondes pulsées pour faire une différence entre les hauteurs des ondes pulsées des impulsions amplifiées, d'où la production d'impulsions classées en groupes par gammes de hauteur d'ondes pulsées,

un compteur d'impulsions pour compter pendant un temps prédéterminé chaque nombre d'impulsions appartenant à des groupes respectifs, et

un ordinateur pour recevoir chaque nombre compté par le compteur d'impulsions, d'où l'obtention d'une image du corps objet par rayonnement, caractérisé en ce que:

les éléments sensibles au rayonnement sont des éléments en semi-conducteur, où l'effet d'absorption photoélectrique est plus grand que l'effet d'absorption Compton.

6. Appareil de radiographie par comptage de quanta classés par énergie selon la revendication 5, dans lequel:

la source de rayonnement émet une ou plusieurs sortes de rayons de rayonnement choisis parmi un rayon X, un faisceau γ, un faisceau β, un faisceau de neutrons et un faisceau de mesons,

chaque rayon de rayonnement peut avoir une sorte d'énergie dans le cas où les rayons de rayonnement sont de plusieurs sortes.

7. Appareil radiographique par comptage de quanta classés par énergie selon la revendication 5, dans lequel:

la source de rayonnement émet une ou plusieurs sortes de rayons de rayonnement choisis parmi un rayon X, un faisceau γ, un faisceau β, un faisceau de neutrons et un faisceau de mesons,

chaque rayon de rayonnement peut avoir une sorte d'énergie dans le cas où le rayonnement est de plusieurs sortes,

la source de rayonnement émet continuellement ou par intermittence les rayons de rayonnement,

la source de rayonnement émet chaque sorte de rayons de rayonnement d'énergie ou chaque sorte de rayons de rayonnement alternativement ou simultanément dans le cas où les rayons de rayonnement sont de plusieurs sortes de rayons de rayonnement ou plusieurs sortes de rayons de rayonnement d'énergie.

FIG.1(a)

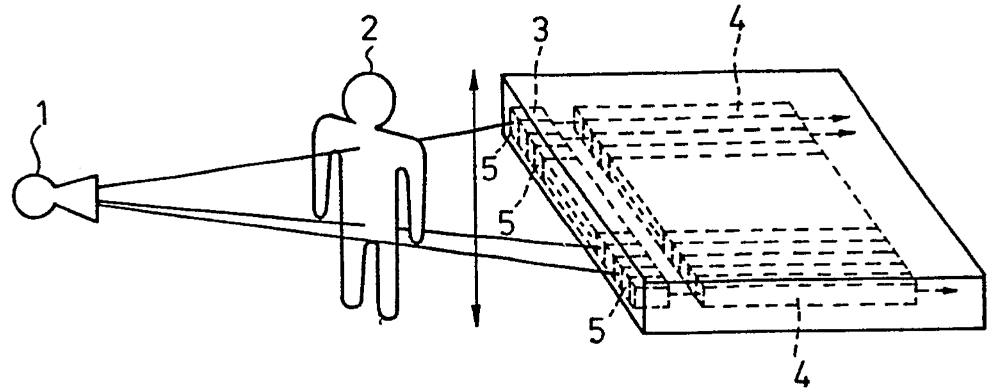


FIG.1(b)

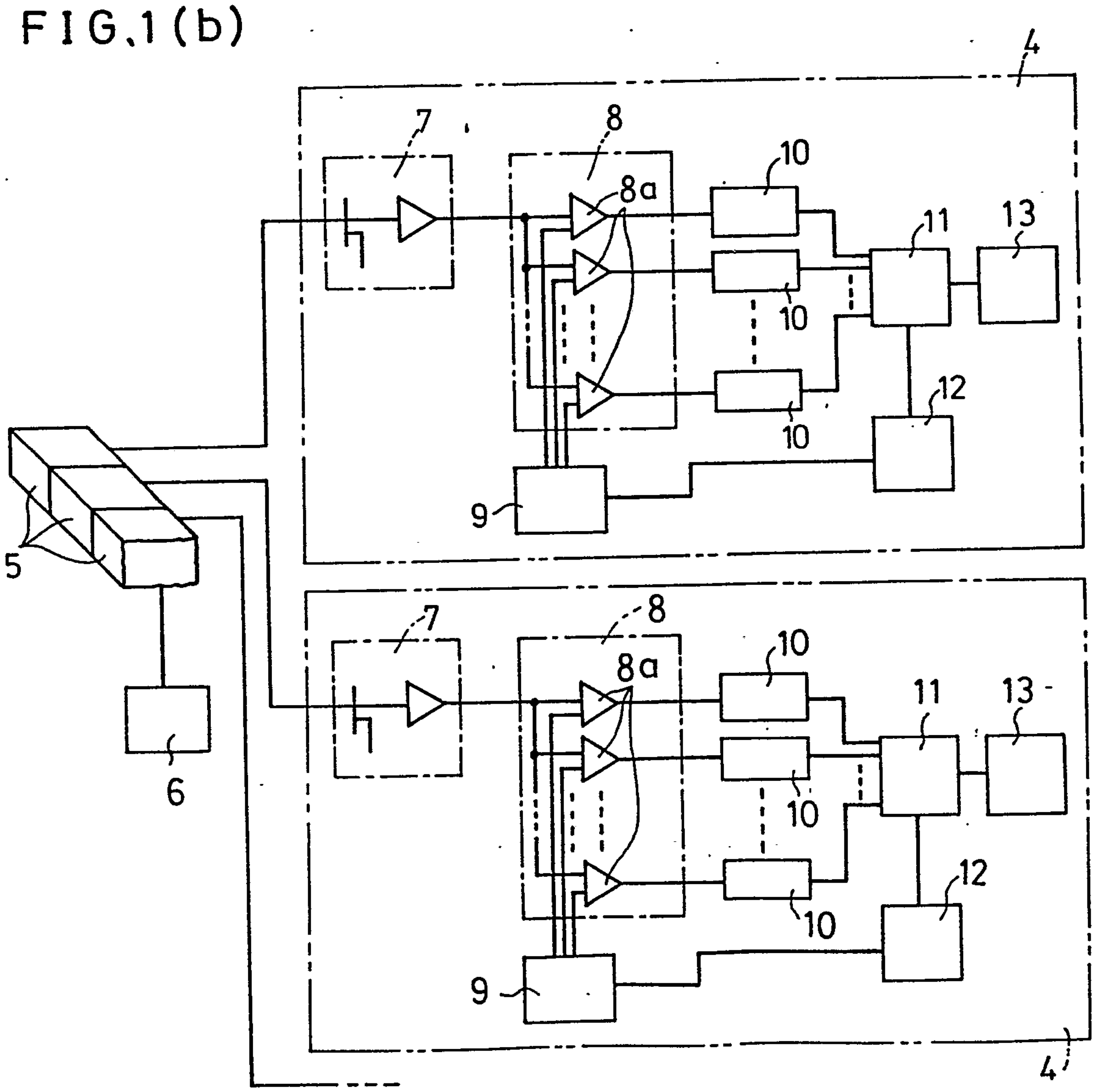

FIG.2 (a)

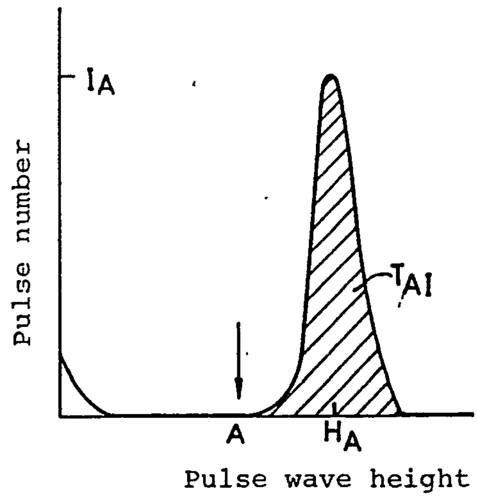

$I_A$
Pulse number
$T_{AI}$
A
$H_A$
Pulse wave height


FIG.2 (b)

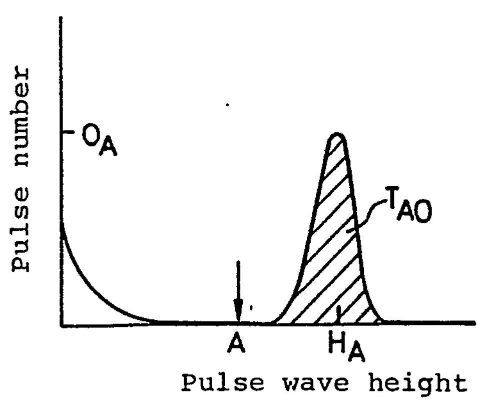

$O_A$
Pulse number
$T_{AO}$
A
$H_A$
Pulse wave height

FIG.3(a)

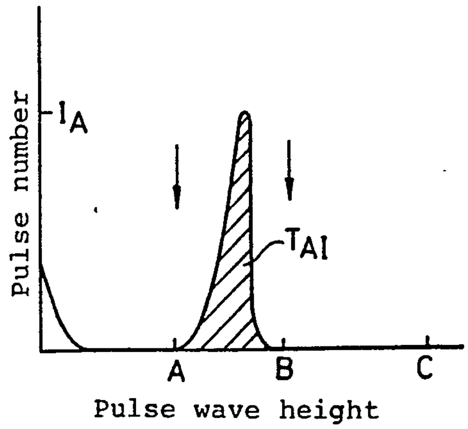


FIG.3(b)

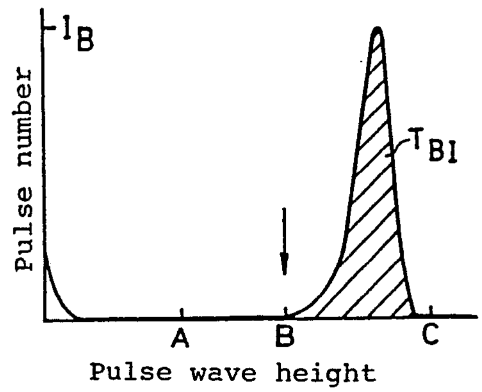


FIG.3(c)

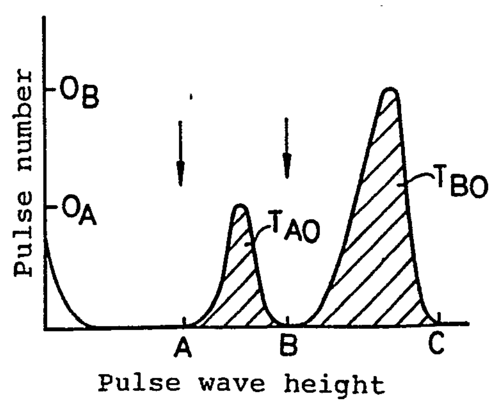

FIG.4

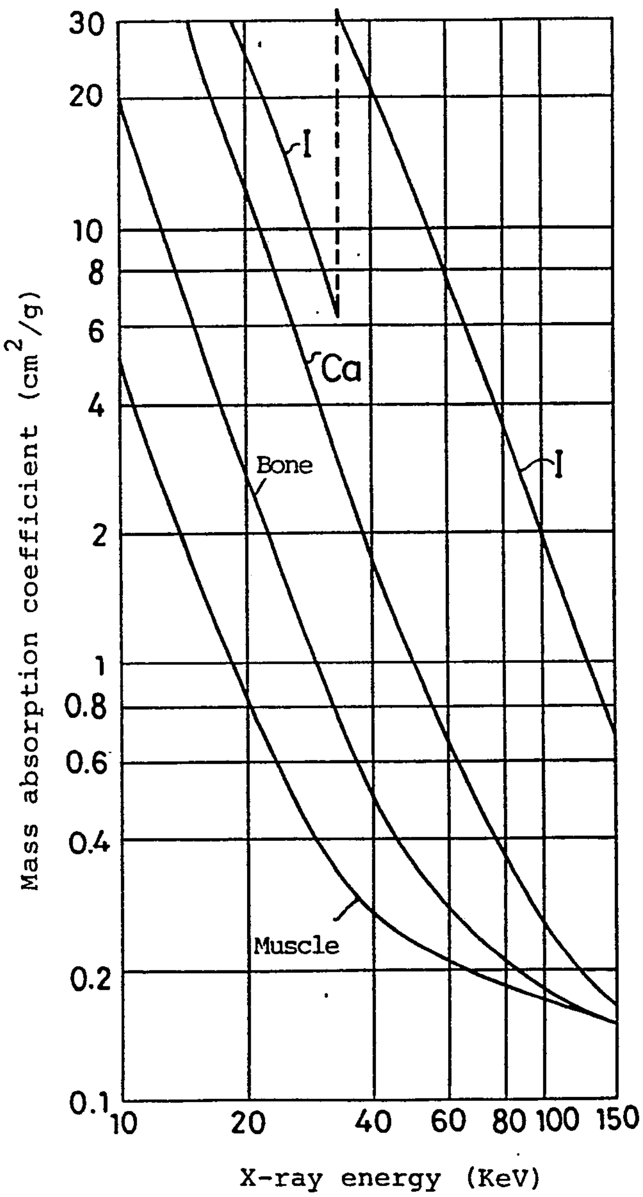
30
20
10
8
6
4
2
1
0.8
0.6
0.4
0.2
0.1
Mass absorption coefficient (cm²/g)
I
Ca
Bone
I
Muscle
10
20
40
60
80
100
150
X-ray energy (KeV)

FIG.5

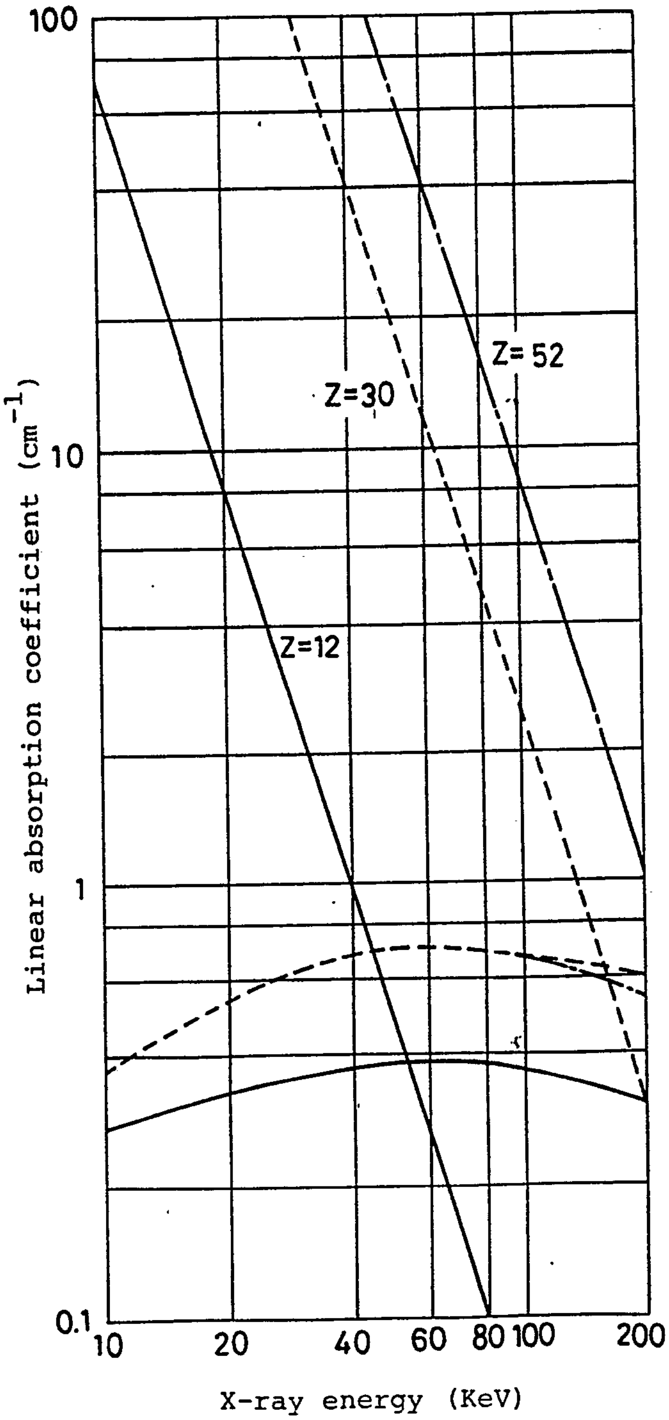
100
10
1
0.1
Linear absorption coefficient (cm$^{-1}$)
Z=52
Z=30
Z=12
10
20
40
60
80
100
200
X-ray energy (KeV)